# NEW EUROPEAN PATENT SPECIFICATION

(11) **EP 0 244 267 B2**
(45) Date of publication and mention of the opposition decision: **01.12.2004**
(45) Mention of the grant of the patent: 17.08.1994
(21) Application number: 87303944.0
(22) Date of filing: 01.05.1987
(51) Int. Cl.: C12N 15/12, C12N 15/62, C07K 14/705, C12N 5/10, C12P 21/00

(54) **Variants of decay accelerating factor (DAF) prepared by recombinant DNA technology**
Varianten des abbaubeschleunigenden Faktors (DAF), hergestellt durch rekombinante DNS-Technologie
Variants du facteur accélérant la décomposition (DAF), préparés par technologie d'ADN recombinant

(30) Priority: 02.05.1986 US 859107
(43) Date of publication of application: 04.11.1987
(73) Proprietor: GENENTECH, INC., South San Francisco California 94080 (US); NEW YORK UNIVERSITY, New York, NY 10012 (US)
(72) Inventor: Caras, Ingrid Wendy, San Francisco, CA 94122 (US); Nussenzweig, Victor, New York, New York 10012 (US); Davitz, Michael A., Riverdale, New York 10463 (US); Martin, David W. Jr., San Francisco, California 94109 (US)
(74) Representative: Armitage, Ian Michael

(56) References cited:
- WO-A-86/07062
- Proceedings of the National Academy of Science USA, vol. 84, April 1987, pages 2007-2011. M.E. Medof et al.: "Cloning and characterization of cDNAs encoding the complete sequence of decay-accelarating factor of human complement"
- Nature, vol. 325, February 5, 1987, pages 545-549, London, GB. I.W. CARAS et al.: "Cloning of decay-accelarating factor suggests novel use of splicing to generate two proteins"
- Journal of Immunology, vol. 129, no. 1, July 1982, pages 184-189, Am. Ass. of Imm. US. A. Nicholson-Weller et al.: "Isolation of a human erytrocyte membrane glycoprotein with decay-accelerating activity for C3 convertases of thecomplement system"
- Immunology Today, vol. 6, no. 6, 1985, pages 188-192, Amsterdam, NL, V. Miachael Holers et al.: "Human C3b- and C4b-regulatory proteins: a new multi-gene family"
- Journal of Experimental Medicine, vol. 160, November 1984, pages 1558-1578; New York, N.Y. US,. M.E. Medof et al.: "Inhibition of complement activation on the surface of cells after incorporation of decay-accelerating factor (DAF) into their membranes"

## Description

This applicatin relates to the preparation of decay accelerating factor (hereinafter abbreviated as DAF) in recombinant cell culture. In particular, it is concerned with the large scale manufacture of DAF suitable for pharmaceutical or diagnostic use.

Antigenic cells targeted by the humoral immune response are lysed by a process called complement activation. This process consists of a series or cascade of proteolytic activities initiated by the binding of antibody with its antigen. The components that participate in complement activation are many and complex, although for the purposes herein the most important are C4b and C3b. In a key step in complement activation, these two proteins become covalently associated with the target cell surface and then serve as anchors for the assembly of C3 and C5 convertases, the amplifying enzymes of the cascade.

Complement activation must focus only on the target and must not occur on host cells. However, in the course of complement activation, large numbers of nascent C4b and C3b fragments are liberated into the fluid phase. Most react with water, but some by chance could bind to nearby host cells and lead to their damage. For this and possibly other reasons, the activities of bound, as well as free, C3b and C4b fragments are under strict control by a complex system of serum and membrane proteins.

Recent evidence (Medof, et al. 1982. "J. Exp. Med." 156:1739; Medof, et al. 1984. "J. Exp. Med." 159:1669) suggests that regulation of the activities of substrate-bound C4b and C3b is distinct from control of the fluid phase fragments. The functions of the former are controlled mainly by two membrane proteins: the C3b/C4b receptor (CR1) and DAF. CR1 dissociates C2 and factor B from C4b and C3b in C3 and C5 convertase complexes and promotes the cleavage of C3b (Medof, et al. 1982. "J. Exp. Med." 156:1739; Fearon, D.T. 1979. "Proc. Natl. Acad. Sci. USA" 76:5867; Medicus, et al. 1983. "Eur. J. Immunol." 13:465; and Ross, et al. 1982 "J. Immunol." 129:2051) and C4b (Medof, et al. 1984. "J. Exp. Med." 159:1669; lida et al. 1981. "J. Exp. Med." 153:1138) by the serum enzyme C3b/C4b inactivator (I). DAF has been shown also to enhance the decay dissociation of C2 and factor B from C3 convertases (Nicholson-Weller, et al. 1982, "J. Immunol." 129:205 and Pangburn, M.K. et al. 1983 "J. Exp. Med." 157:1971) . The reason for the apparent redundancy in regulatory activities of the two membrane factors and their respective roles in convertase control has remained unclear. Abnormalities of CR1 have been found in systemic lupus erythematosus (SLE) (Miyakawa, Y. et al. 1981 "Lancet" 2:493; lida, K. et al. 1982 "J. Exp. Med." 155:1427; Wilson, J. G. et al. 1982 "N. Engl. J. Med." 307:981; Taylor, R.P. et al. 1983 "Arthritis Rheum." 26:736), a condition associated with defective immune complex handling, and abnormalities of DAF have been found in paroxysmal nocturnal hemoglobinuria (PNH) (Pangburn, M.K. et al. 1983 "J. Exp. Med." 157:1971; Pangburn, M.K. et al. 1983 "Proc. Natl. Acad. Sci." 80:5430; Nicholson-Weller, A. et al. 1983 "Proc. Natl. Acad. Sci." 80:5066), a condition associated with heightened susceptibility of blood cells to lysis.

DAF was reported to have been purified to a single 70 Kd band on silver stained SDS-PAGE from a pooled extract of human erythrocytes stroma (Medof et al., 1984, "J. Exp. Med." 160:1558). The molecule was hydrophobic and tended to form multimers of ≧ 150 Kd as determined by molecular sieve chromatography. Purified DAF could reassociate with red blood cells. Only a small number of DAF molecules (<100) had a significant effect on the hemolytic effect of activated complement. Medof et al. concluded that DAF can only function intrinsically within the cell membrane, and suggested that it offered the possibility of correcting in vitro the defect in the membranes of cells from patients with PNH.

Existing methods for obtaining DAF are unsatisfactory for its commercial preparation. Red cells contain extremely small quantities of DAF. Furthermore, blood contains viruses and other biologically active components which pose a risk of adverse reactions in recipients or users.

Red blood cell DAF is limited to the native membrane bound form, including any naturally occurring alleles as may exist. Methods are needed for synthesizing amino acid and glycosylation variants which can function as DAF agonists or antagonists, or which will exhibit other desirable characteristics such as the absence of C-Terminal lipid, resistance to proteases, or the ability to deliver DAF to the membranes of target cells.

Accordingly, it is an object herein to prepare DAF in commercial quantity from a therapeutically acceptable source.

It is a further object to obtain human DAF from a source that is completely uncontaminated with other human proteins.

It is an additional object to prepare amino acid sequence and glycosylation variants of DAF.

Other objects of this invention will be apparent from the specification as a whole.

### Summary

The objects of this invention can be accomplished by expression of DAF in recombinant cell culture, a process that fundamentally comprises providing nucleic acid encoding DAF, transforming a host cell with the DAF-encoding nucleic acid, and culturing the cell in order to express DAF in the host cell culture.

The method of this invention enables the preparation of novel forms of DAF. Including amino acid sequence variants and glycosylatin variants. Amino acid sequence variants consist of deletions, substitutions and insertions of one or more DAF amino acid residues DAF also is expressed in a form unaccompanied by the glycosylation associated with native DAF (including unaccompanied by any glycosylation whatsoever), obtained as a product of expression of DAF in heterologous recombinant cell culture.

Unexpectedly, we discovered during studies of cell processing of DAF mRNA that the membrane-bound form of DAF (mDAF) is not the only form in which it is expressed in vivo. In fact another form of DAF exists, called sDAF. This form is encoded by an mRNA species from which the last 3 intron has not been spliced, resulting in an amino acid sequence C-terminal to residue 327 that is entirely different from that of mDAF. The novel C-terminus of sDAF is believed to result in vivo in the secretion of the protein into the blood stream, where it is biologically active, because the presence of the intron changes the reading frame of the last exon so as to eliminate the region to which phosphatidylinositol (the membrane anchor for mDAF) is bound. This novel form of DAF was unappreciated until the pioneering work herein was accomplished, and it differs from mDAF in containing an antigenically distinct C-terminus. sDAF is useful in diagnosis of PNH since it is now possible to determine whether the condition in an individual results from a failure to express any of the DAF gene or a failure of post-translational processing to attach the phosphatidylinositol anchor.

Novel nucleic acids also are provided, including (1) cell free nucleic acid identified as encoding DAF, including genomic DNA, cDNA or RNA, (2) DNA encoding DAF free of an untranslated intervening sequence (introns) or flanking genomic DNA, and (3) nucleic acid encoding DAF which is free of nucleic acid encoding any other protein homologous to the source of the nucleic acid that encodes DAF. Also within the scope of this invention is nucleic acid which does not encode DAF but which is capable of hybridizing with nucleic acid encoding DAF.

Nucleic acid encoding DAF is useful in the expression of DAF in recombinant cell culture or for assaying test samples for the presence of DAF-encoding nucleic acid. Labelled DAF-encoding or hybridizing nucleic acid is provided for use in such assays.

Recombinant DAF is formulated into therapeutically acceptable vehicles and administered for the treatment of PNH or inflammatory or cell lytic autoimmune diseases. DAF conjugates or fusions are prepared that deliver DAF to target cells in order to inhibit complement activation at the surfaces of such cells. The conjugates or fusions are useful for ameliorating allograft rejection or autoimmune diseases.

### Brief Description of the Drawing

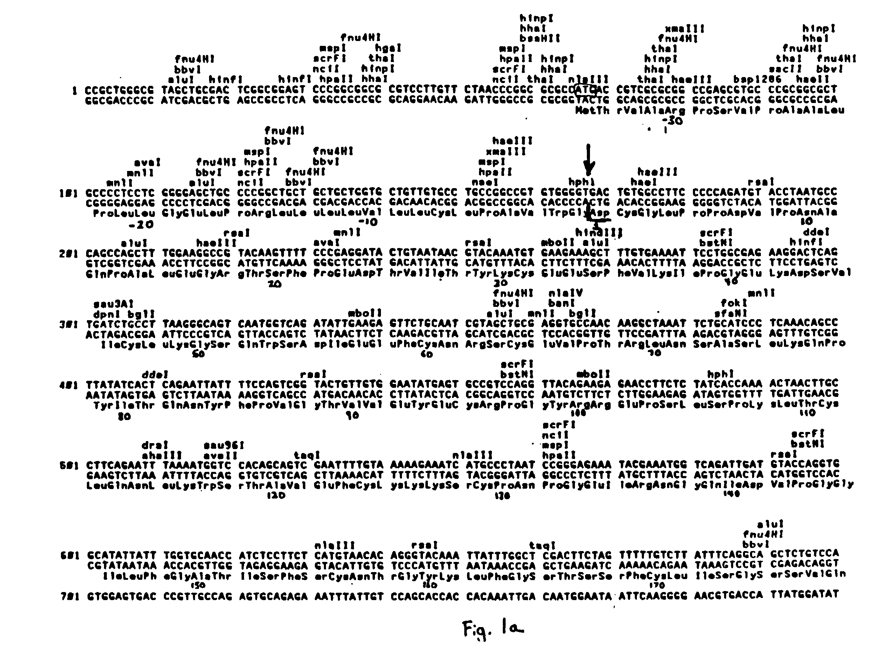

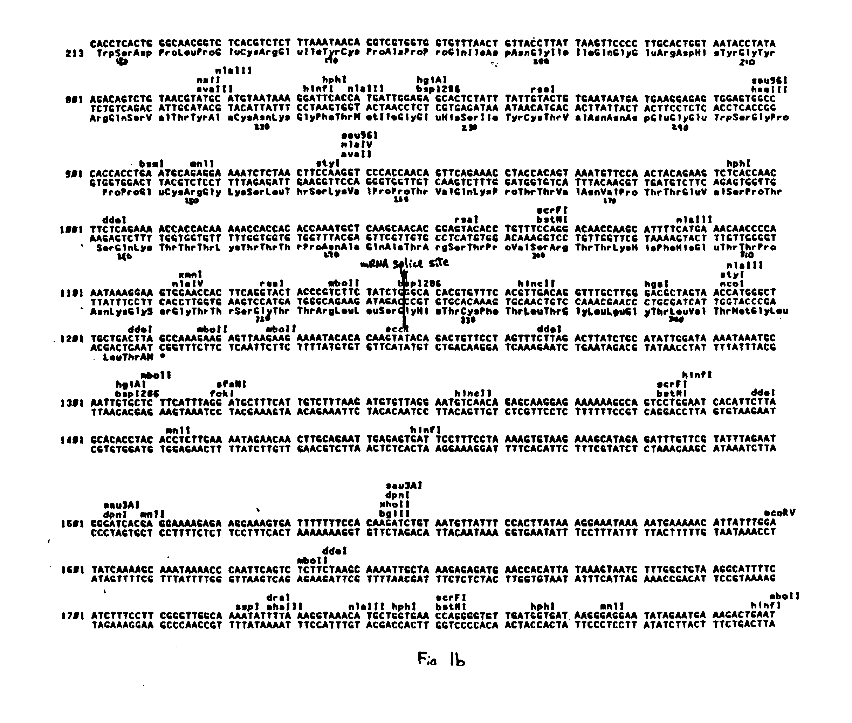

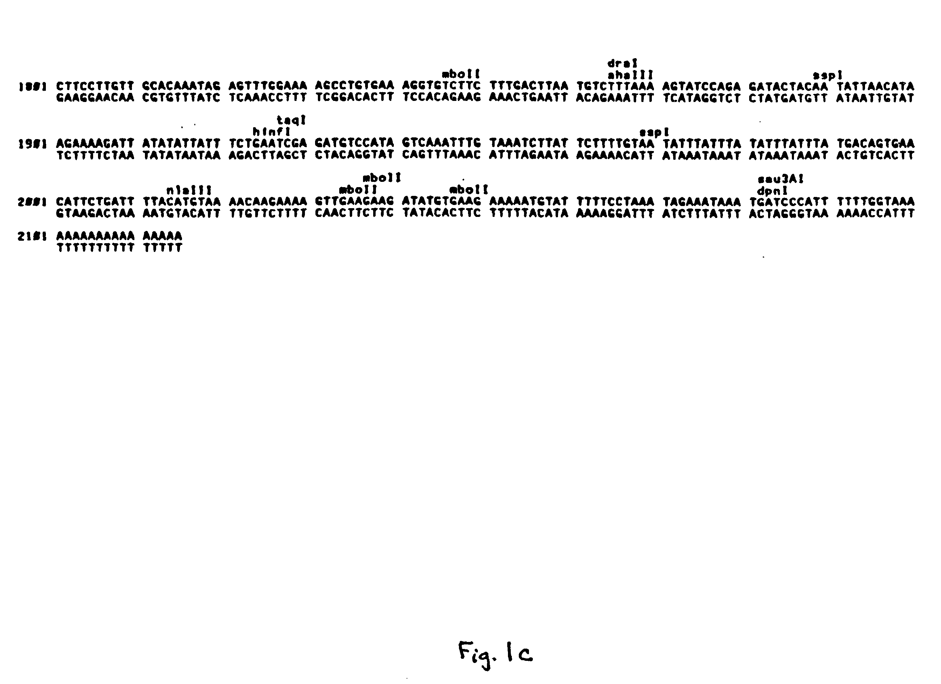

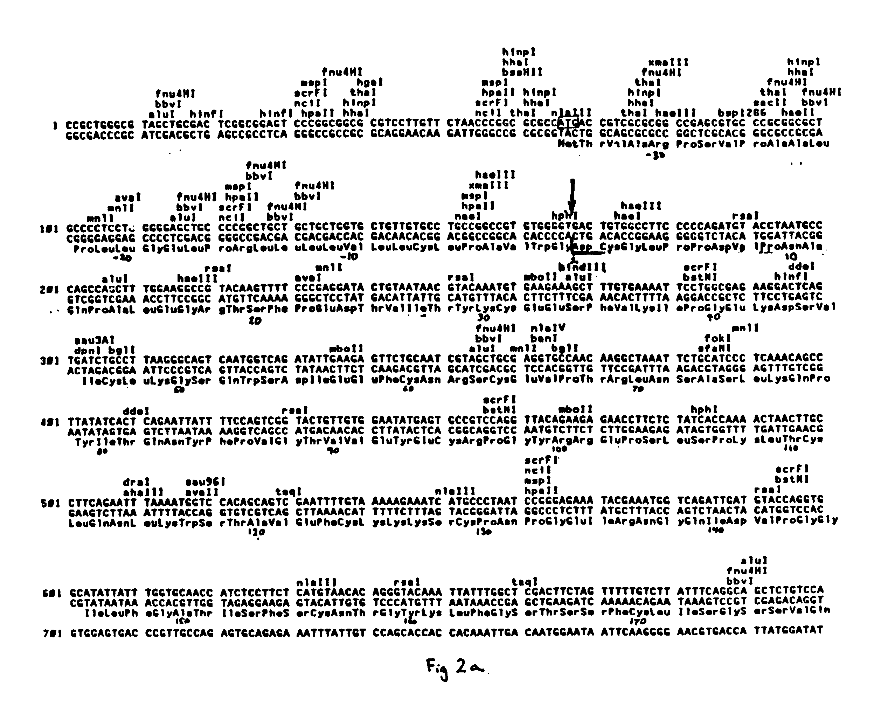

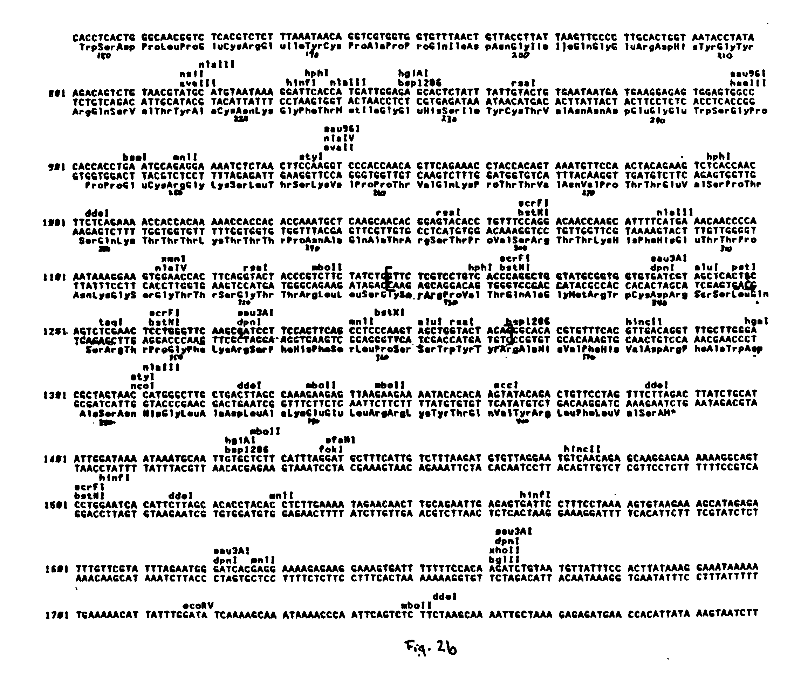

Figs. 1a - 1c depict the cDNA sequence for clones λ33 (to the Hind III site at residue 1) and λ47 (Hind III to the 3' end). The point at which the intron is removed is designated by an asterisk. The probable phosphatidylinositol derivatization site is Cys₃₃₀ and the putative transmembrane region extends from residue 331-347. Amino and residues are numbered from the mature amino terminus at Asp¹. Figs. 2a - 2c depict the cDNA sequence of clones λ33 to the Hind III site at residue +1) and λ41 (Hind III to 3'end) encoding human sDAF. The unspliced intron in the cDNA encoding sDAF is bracketed. Restriction enzyme sites are shown using convention abbreviations. The imputed amino acid sequence for each DAF species is shown, together with the secretory leader and mature N-terminus of each (designated by arrows).

### Detailed Description

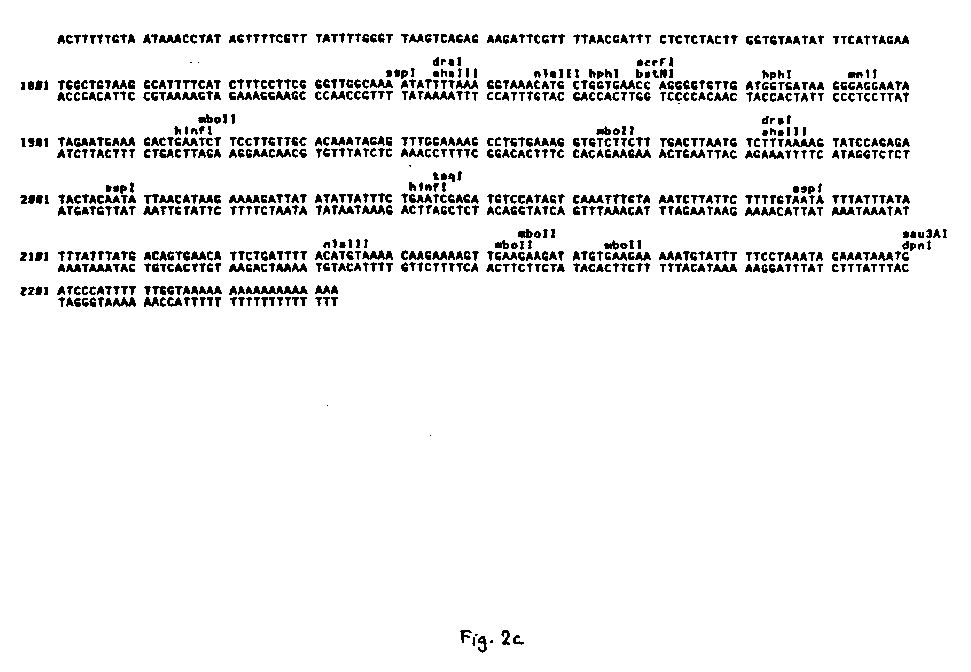

DAF is defined to be any molecule having the pre or mature amino acid sequence set forth in Figs. 1 or 2 as well as their amino acid sequence or glycosylation variants (including natural alleles) which are capable of exhibiting a biological activity in common with the native DAF of Figs. 1 or 2. Henceforth, the term DAF shall mean either or both forms unless otherwise appropriate. Native DAF is DAF obtained from serum, blood cells or other animal fluids or tissues. DAF biological activity is defined as any of 1) immunological cross-reactivity with at least one epitope of native DAF, or 2) the possession of at least one hormonal, regulatory or effector function qualitatively in common with native DAF. Since amino acid sequence variations of DAF having antagonist or agonist activity are included, an amino acid sequence variant need not exhibit any DAF immunomodulatory activity to fall within the definition of DAF. For example, a variant may act as an antagonist and competitively inhibit native DAF, yet have no immunomodulatory activity per se. Alternatively, the variant may be neither an antagonist nor have immunomodulatory activity, but still fall within the definition if it remains capable of cross-reacting with antibody raised against native DAF. An example of a presently known DAF immunomodulatory activity is inhibition of C4b2a functional activity (Medof et al., 1984, Id.).

Amino acid sequence variants of DAF include deletions from, or insertions or substitutions of residues within the pre or mature DAF sequence shown in Figs. 1 or 2. Amino acid sequence deletions generally range about from 1 to 10 residues and typically are contiguous. Contiguous deletions ordinarily are made in even numbers of residues, but single or odd numbers of deletions are within the scope hereof. Representative deletions are [des Cys₃₃₀ ] mature mDAF, [des Cys₃₃₀ - Thr₃₄₇ ] mature mDAF, [des Thr₂ - Gly₃₂₇ ] mature sDAF. A particularly interesting deletion is that of Cys₃₃₀ - Thr₃₄₇ from mDAF. This eliminates the membrane anchor site and transmembrane region, resulting in a molecule that, like sDAF, is secreted but which bears none of the unique antigenic determinants of sDAF.

Insertions also are preferably made in even numbers of residues when the variation falls within the mature DAF sequence, although insertions may range about from 1 to 5 residues in general. However, insertions also include fusions onto the amino or carboxyl termini of DAF of from 1 residue to polypeptides of essentially unrestricted length. An example of a single terminal insertion is mature DAF having an N-terminal methionyl. This variant is an artifact of the direct expression of DAF in recombinant cell culture, i.e., expression without a signal sequence to direct the secretion or cell membrane assocation of mature DAF. Other examples of terminal insertions include 1) fusions of heterologous signal sequences to the N-terminus of mature DAF in order to facilitate the secretion of mature DAF from recombinant hosts, 2) fusions of immunogenic polypeptides, e.g. bacterial polypeptides such as beta-lactamase or an enzyme encoded by the E. coli trp locus and 3) fusions with cell surface binding substances, including hormones, growth factors or antibodies. Fusions with cell surface binding substances need not be produced by recombinant methods, but can be the product of covalent or noncovalent association with DAF, including its phosphatidylinositol group. For example, an antibody or fragment thereof bearing the variable region is covalently bound to, or expressed in recombinant cell culture as a fusion with, the C-terminus of DAF. For amelioration of allograft rejection the DAF is bound to antibodies specific for the HLA antigens of the allograft. The antibody and DAF are covalently bonded, for example, by the method of EP 170,697A, although other methods for linking proteins to antibodies are conventional and known to the artisan. Immunogenic fusions are useful for preparing immunogenic DAFs suitable as vaccines for preparing anti-DAF antibodies. These are useful for the preparation of diagnostic reagents. Representative insertions are [Thr₃₂₉ LeuLeu Cys₃₃₀] mature DAF, [Arg₁₀₀ His Arg₁₀₀] mature DAF, [Lys₁₂₅ GlnLys₁₂₆ GlnLys₁₂₇] mature DAF, [Pro₁₉₃LeuLeu Ala₁₉₄] mature DAF, [Pro₂₄₇ AspAspGlu₂₄₈] mature DAF, [Thr₂₈₂SerSerThr₂₈₃] mature DAF, and [Gly₃₁₆ ThrThrThr₃₁₇] mature DAF.

The third group of variants are those in which at least one residue in the DAF molecule has been removed and a different residue inserted in its place. Such substitutions generally are made in accordance with the following Table.

**Table 1**

| Original Residue | Exemplary Substitutions |
|---|---|
| Ala | gly; ser |
| Arg | lys |
| Asn | gln; his |
| Asp | glu |
| Cys | ser |
| Gln | asn |
| Glu | asp |
| Gly | ala |
| His | asn; gln |
| Ile | leu; val |
| Leu | ile; val |
| Lys | arg; gln; glu |
| Met | leu; ile |
| Phe | met; leu; tyr |
| Ser | thr |
| Thr | ser |
| Trp | tyr |
| Tyr | trp;phe |
| Val | ile; leu |

Substantial changes in function or immunological identity are made by selecting substitutions that are less conservative than those in Table 1, i.e., selecting residues that differ more significantly in their effect on maintaining (a) the structure of the polypeptide backbone in the area of the substitution, for example as a sheet or helical conformation, (b) the charge or hydrophobicity of the molecule at the target site or (c) the bulk of the side chain. The substitutions in general expected to produce the greatest changes in DAF properties will be those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having such a side chain, e.g. glycine.

Representative substituted DAFs are [Cys₃₃₀ -> Met ] mature mDAF, [Cys₃₃₀ ->Ser] mature mDAF, [Cys₂ -> Ser] mature DAF, [Lys₁₂₅ Lys₁₂₆ -> Gln] mature DAF, [Gly₁₄₄ -> Pro] mature DAF, [Ile₁₄₆ -> Met] mature DAF, [Phe₁₆₉ -> Tyr] mature DAF, [Pro₁₉₂ -> Gly] mature DAF, [Ile₂₀₁ -> Leu] mature DAF, [Asn₂₃₆Asn₂₃₇ -> AspAsp] mature DAF, [Glu₂₃₉ -> Asp] mature DAF, [Ser₂₅₆ -> Tyr] mature DAF, [Val₂₆₈ - > Phe] mature DAF, [Lys₂₈₅ -> Gln] mature DAF, [Thr₂₉₄ -> Ser] mature DAF and [Leu₃₂₄ -> Ser] mature DAF.

The above described variants are made in either sDAF or mDAF. The following variants are made in the unique sDAF C-terminal:
[Lys₃₅₂ -> Gln] mature sDAF, [Cys₃₃₉ -> Ser] mature sDAF, [Arg₃₉₄ -> His] mature sDAF and mature sDAF [Leu₄₀₃ Phe₄₀₄ Leu₄₀₅ -> SerTyrSer] mature sDAF.

For the purposes herein, any naturally occurring alleles are not included within the scope of DAF variants because the variants described herein are predetermined DAF variants.

The C-terminal domain of mDAF contains a site to which lipid is attached in the course of post-translational processing. This domain or any fragment of mDAF containing it, is expressed as a fusion with any other polypeptide for which it is desired to create a membrane-bound form. For example, an ordinarily secreted hormone is expressed in recombinant cell culture as a C-terminal fusion of the preprotein with the C-terminal domain of mDAF. Rather than being secreted this fusion will be transported to the cell membrane and remain lodged there by virtue of the phosphatidycholine anchor. Such recombinant cells are useful as immunogens or vaccines for the hormone or other selected polypeptide. Sequestering the polypeptide in the membrane also protects it from dilution into the culture medium. Finally, fusion polypeptides having C-terminal lipids are useful in diagnostic assays for the polypeptides or their antibodies since the terminal lipid provides a convenient site for adsorption onto immobilizing matrices such as alkyl sepharose, polyolefin microtiter or test tube surfaces and the like.

Most deletions and insertions, and substitutions in particular, will not produce radical changes in the characteristics of the DAF molecule. However, when it is difficult to predict the exact effect of the substitution, deletion or insertion in advance of doing so, for example when modifying the DAF receptor binding domain or an immune epitope, one skilled in the art will appreciate that the effect will be evaluated by routine screening assays. For example, a variant typically is made by site specific mutagenesis of the native DAF-encoding nucleic acid, expression of the variant nucleic acid in recombinant cell culture and, optionally, purification from the cell culture for example by immunoaffinity adsorption on a rabbit polyclonal anti-DAF column (in order to adsorb the variant by at least one remaining immune epitope). The activity of the cell lysate or purified DAF variant is then screened in a suitable screening assay for the desired characteristic. For example, a change in the immunological character of the DAF, such as affinity for a given antibody, is measured by a competitive-type immunoassay. Changes in immunomodulator activity are measured by the C4b2a assay, although as more becomes known about the functions in vivo of sDAF and mDAF other assays will become useful in such screening. Modifications of such protein properties as redox or thermal stability, hydrophobicity, susceptibility to proteolytic degradation, or the tendency to aggregate with carriers or into multimers are assayed by methods well known to the artisan.

DAF preferably is made by synthesis in recombinant cell culture. In order to do so, it is first necessary to secure nucleic acid that encodes DAF. The inventors encountered considerable hardship in attempting to identify any nucleic acid encoding DAF. The sequence of the human mDNA encoding DAF that was ultimately determined is shown in Fig. 1. As noted above, study of cDNAs from hela cells led to the identification of cDNA encoding sDAF, shown in Fig. 2. Once this DNA has been identified it is a straight-forward matter for those skilled in the art to obtain it by nucleic acid hybridization to genomic libraries of human DNA or, .if it is desired to obtain DNA encoding the DAF of another animal species, then by hybridization of DNA libraries from cells of that species. The hybridization analysis is now straight-forward because Figs. 1 and 2 enable the preparation of very long synthetic probes that are perfect or nearly perfect matches for the target DNA.

It is possible that the cDNA or genomic library selected as the source for the DAF nucleic acid will not contain a single clone encoding the full length DAF, only partial clones. These partial clones and fragments are readily assembled into a full length DNA by cleaving the partial clones at selected restriction sites in overlapping sections, recovering each of the desired fragments and ligating them in the proper order and orientation. If necessary, oligonucleotides are prepared to supply any missing sequences.

The DAF-encoding nucleic acid is then ligated into a replicable vector for further cloning or for expression. Vectors are useful for performing two functions in collaboration with compatible host cells (a host-vector system). One function is to facilitate the cloning of the nucleic acid that encodes the DAF, i.e., to produce usable quantities of the nucleic acid. The other function is to direct the expression of DAF. One or both of these functions are performed by the vector-host system. The vectors will contain different components depending upon the function they are to perform as well as the host cell that is selected for cloning or expression.

Each vector will contain nucleic acid that encodes DAF as described above. Typically, this will be DNA that encodes the DAF in its mature form linked at its amino terminus to a secretion signal. This secretion signal preferably is the DAF presequence that normally directs the secretion of DAF from human cells in vivo. However, suitable secretion signals also include signals from other animal DAFs, viral signals or signals from secreted polypeptides of the same or related species.

Expression and cloning vectors contain a nucleic acid sequence that enables the vector to replicate in one or more selected host cells. Generally, in cloning vectors this sequence is one that enables the vector to replicate independently of the host chromosomes, and includes origins of replication or autonomously replicating sequences. Such sequences are well-known for a variety of bacteria, yeast and viruses. The origin of replication from the well-known plasmid pBR322 is suitable for most gram negative bacteria, the 2µ plasmid origin for yeast and various viral origins (SV40, polyoma, adenovirus, VSV or BPV) are useful for cloning vectors in mammalian cells. Origins are not needed for mammalian expression vectors (the SV40 origin is used in the Examples only because it contains the early promoter). Most expression vectors are "shuttle" vectors, i.e. they are capable of replication in at least one class of organisms but can be transfected into another organism for expression. For example, a vector is cloned in E. coli and then the same vector is transfected into yeast or mammalian cells for expression even though it is not capable of replicating independently of the host cell chromosome.

DNA also is cloned by insertion into the host genome. This is readily accomplished with bacillus species, for example, by including in the vector a DNA sequence that is complementary to a sequence found in bacillus genomic DNA. Transfection of bacillus with this vector results in homologous recombination with the genome and insertion of DAF DNA. However, the recovery of genomic DNA encoding DAF is more complex than that of an exogenously replicated vector because restriction enzyme digestion is required to excise the DAF DNA.

Generally, DNA is inserted into a host genome for purposes of preparing a stable cell line or microbe for DAF expression.

Expression and cloning vectors should contain a selection gene, also termed a selectable marker. This is a gene that encodes a protein necessary for the survival or growth of a host cell transformed with the vector. The presence of this gene ensures that any host cell which deletes the vector will not obtain an advantage in growth or reproduction over transformed hosts. Typical selection genes encode proteins that (a) confer resistance to antibiotics or other toxins, e.g. ampicillin, neomycin, methotrexate or tetracycline, (b) complement auxotrophic deficiencies, or (c) supply critical nutrients not available from complex media, e.g. the gene encoding D-alanine racemase for bacilli.

A suitable selection gene for use in yeast is the trp1 gene present in the yeast plasmid YRp7 (Stinchcomb et al., 1979, "Nature", 282: 39; Kingsman et al., 1979, "Gene", 7: 141; or Tschemper et al., 1980, "Gene", 10: 157). The trp1 gene provides a selection marker for a mutant strain of yeast lacking the ability to grow in tryptophan, for example ATCC No. 44076 or PEP4-1 (Jones, 1977, "Genetics", 85: 12). The presence of the trp1 lesion in the yeast host cell genome then provides an effective environment for detecting transformation by growth in the absence of tryptophan. Similarly, Leu2 deficient yeast strains (ATCC 20,622 or 38,626) are complemented by known plasmids bearing the Leu2 gene.

Examples of suitable selectable markers for mammalian cells are dihydrofolate reductase (DHFR) or thymidine kinase. Such markers enable the identification of cells which were competent to take up the DAF nucleic acid. The mammalian cell transformants.are placed under selection pressure which only the transformants are uniquely adapted to survive by virtue of having taken up the marker. Selection pressure is imposed by culturing the transformants under conditions in which the concentration of selection agent in the medium is successively changed, thereby leading to amplification of both the selection gene and the DNA that encodes DAF. Amplification is the process by which genes in greater demand for the production of a protein critical for growth are reiterated in tandem within the chromosomes of successive generations of recombinant cells. Increased quantities of DAF are synthesized from the amplified DNA.

For example, cells transformed with the DHFR selection gene are first identified by culturing all of the transformants in a culture medium which lacks hypoxanthine, glycine, and thymidine. An appropriate host cell in this case is the Chinese hamster ovary (CHO) cell line deficient in DHFR activity, prepared and propagated as described by Urlaub and Chasin, 1980, "Proc. Nat'l. Acad. Sci. USA" 77: 4216. A particularly useful DHFR is a mutant DHFR that is highly resistant to MTX (EP 117,060A). This selection agent can be used with any otherwise suitable host, e.g. ATCC No. CCL61 CHO-K1), notwithstanding the presence of endogenous DHFR. The DHFR and DAF-encoding DNA then is amplified by exposure to an agent (methotrexate, or MTX) that inactivates the DHFR. One ensures that the cell requires more DHFR (and consequently amplifies all exogenous DNA) by selecting only for cells that can grow in successive rounds of ever-greater MTX concentration.

Other methods, vectors and host cells suitable for adaptation to the synthesis of the hybrid receptor in recombinant vertebrate cell culture are described in M.J. Gething et al., "Nature" 293: 620-625 (1981); N. Mantei et al., "Nature" 281: 40-46; and A. Levinson et al., EP 117,060A and 117,058A. A particularly useful starting plasmid for mammalian cell culture expression of DAF is pE342.HBV E400.D22 (also called pE348HBVE400D22, EP 117,058A).

Expression vectors, unlike cloning vectors, should contain a promoter which is recognized by the host organism and is operably linked to the DAF nucleic acid. Promoters are untranslated sequences located upstream from the start codon of a structural gene (generally within about 100 to 1000 bp) that control the transcription and translation of nucleic acid under their control. They typically fall into two classes, inducible and constitutive. Inducible promoters are promoters that initiate increased levels of transcription from DNA under their control in response to some change in culture conditions, e.g. the presence or absence of a nutrient or a change in temperature. At this time a large number of promoters recognized by a variety of potential host cells are well known. These promoters are operably linked to DAF-encoding DNA by removing them from their gene of origin by restriction enzyme digestion, followed by insertion 5' to the start codon for DAF. This is not to say that the genomic DAF promoter is not usable. However, heterologous promoters generally will result in greater transcription and higher yields of expressed DAF.

Nucleic acid is operably linked when it is placed into a functional relationship with another nucleic acid sequence. For example, DNA for a presequence or secretory leader is operably linked to DNA for a polypeptide if it is expressed as a preprotein which participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, operably linked means that the DNA sequences being linked are contiguous and, in the case of a secretory leader, contiguous and in reading phase. Linking is accomplished by ligation at convenient restriction sites. If such sites do not exist then synthetic oligonucleotide adaptors or linkers are used in accord with conventional practice.

Promoters suitable for use with prokaryotic hosts include the β-lactamase and lactose promoter systems (Chang et al., 1978, "Nature", 275: 615; and Goeddel et al., 1979, "Nature", 281: 544), alkaline phosphatase, a tryptophan (trp) promoter system (Goeddel 1980, "Nucleic Acids Res." 8: 4057 and EPO Appln. Publ. No. 36,776) and hybrid promoters such as the tac promoter (H. de Boer et al., 1983, "Proc. Nat'l. Acad. Sci. USA" 80: 21-25). However, other known bacterial promoters are suitable. Their nucleotide sequences have been published, thereby enabling a skilled worker operably to ligate them to DNA encoding DAF (Siebenlist et al., 1980, "Cell" 20: 269) using linkers or adaptors to supply any required restriction sites. Promoters for use in bacterial systems also will contain a Shine-Dalgarno (S.D.) sequence operably linked to the DNA encoding DAF.

Suitable promoting sequences for use with yeast hosts include the promoters for 3-phosphoglycerate kinase (Hitzeman et al., 1980, "J. Biol. Chem.", 255: 2073) or other glycolytic enzymes (Hess et al., 1968, "J. Adv. Enzyme Reg.", 7: 149; and Holland, 1978, "Biochemistry", 17: 4900), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in R. Hitzeman et al., EP 73,657A. Yeast enhancers also are advantageously used with yeast promoters.

DAF transcription from vectors in mammalian host cells is controlled by promoters obtained from the genomes of viruses such as polyoma, cytomegalovirus, adenovirus, retroviruses, hepatitis-B virus and most preferably Simian Virus 40 (SV40), or from heterologous mammalian promoters, e.g. the actin promoter. The early and late promoters of the SV40 virus are conveniently obtained as an SV40 restriction fragment which also contains the SV40 viral origin of replication (Fiers et al., 1978, "Nature", 273: 113). Of course, promoters from the host cell or related species also are useful herein.

Transcription of DAF-encoding DNA by higher eukaryotes is increased by inserting an enhancer sequence into the vector. An enhancer is a nucleotide sequence, usually about from 10-300 bp, that acts on a promoter to increase its transcription and does so in a manner that is relatively orientation and position independent. Many enhancer sequences are now known from mammalian genes (globin, elastase, albumin, α-fetoprotein and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenoviral enhancers. The enhancer may be spliced into the vector at a position 5' or 3' to the DAF-encoding sequence, but is preferably located at a site 5' from the promoter.

Expression vectors used in eukaryotic host cells (yeast, fungi, insect, plant, animal, human or nucleated cells from other multicellular organisms) will also contain sequences necessary for the termination of transcription and for stabilizing the mRNA. Such sequences are commonly available from the 5' and, occasionally 3' untranslated regions of eukaryotic or viral DNAs or cDNAs. These regions contain regions that are transcribed as polyadenylated segments in the untranslated portion of the mRNA encoding DAF. The 3' untranslated regions also include transcription termination sites.

Suitable host cells for cloning or expressing the vectors herein are prokaryotes, yeast or higher eukaryotic cells. Prokaryotes include gram negative or gram positive organisms, for example E. coli or bacilli. A preferred cloning host is E. coli 294 (ATCC 31,446) although other gram negative or gram positive prokaryotes such as E. coli B, E. coli X1776 (ATCC 31,537), E. coli W3110 (ATCC 27,325), pseudomonas species, or Serratia Marcesans are suitable.

In addition to prokaryotes, eukaryotic microbes such as filamentous fungi or yeast are suitable hosts for DAF-encoding vectors. Saccharomyces cerevisiae, or common baker's yeast, is the most commonly used among lower eukaryotic host microorganisms. However, a number of other genera, species and strains are commonly available and useful herein.

The preferred host cells for the expression of DAF are cells derived from multicellular organisms. DAF's large size, together with its intramolecular disulfide bond(s) and, in the case of mDAF, its unique post-translational processing, suggests that the host cell will optimally be of a higher phylogenetic order than the microbes if one is to expect the recombinant protein to demonstrate optimal conformational fidelity to native DAF. In addition, it may be desirable to glycosylate DAF. All of these functions can be best performed by higher eukaryotic cells. In principle, any higher eukaryotic cell culture is workable, whether from vertebrate or invertebrate culture, although cells from mammals such as humans are preferred. Propagation of such cells in culture is per se well known. See Tissue Culture, Academic Press, Kruse and Patterson, editors (1973). Examples of useful mammalian host cell lines are VERO and HeLa cells, Chinese hamster ovary cell lines, the WI38, BHK, COS-7, MDCK cell lines and human embryonic kidney cell line 293.

Host cells are transformed with the above-described expression or cloning vectors and cultured in conventional nutrient media modified as is appropriate for inducing promoters or selecting transformants containing amplified genes. The culture conditions, such as temperature, pH and the like, suitably are those previously used with the host cell selected for cloning or expression, as the case may be, and will be apparent to the ordinary artisan.

sDAF preferably is recovered from the culture medium as a secreted protein, although it also may be recovered from host cell lysates when directly expressed without a secretory signal. As a first step, the culture medium or lysate is centrifuged to remove particulate cell debris. DAF also is purified from contaminant soluble proteins for example by adsorption on a section column, e.g. ConA, election, adsorption on an anti-sDAF or anti-mDAF immunoaffinity column and elution therefrom. Alternatively, other processes such as chromatography on alkyl Sepharose, silica or an anion or cation exchange resin or gel electrophoresis are used to separate the sDAF from contiminants. mDAF is recovered from transformant cell membranes using the method of Medof et al. (1984. Id.). mDAF variants in which the hydrophobic transmembrane region and/or the mDAF phosphatidylinositol-binding residue are deleted or substituted are recovered in the same fashion as sDAF, although variants in which the transmembrane region remains intact also are recovered from transformant cell membranes.

Since native DAF has a tendency to aggregate under some conditions it may be useful to stabilize the aggregative state of the multimers by providing in the separations a minor amount of a nonionic surfactant such as Tween or polyethylene glycol. A protease inhibitor such as PMSF also may be useful to inhibit proteolytic degradation during purification, and antibiotics may be included to prevent the growth of adventitious contaminants.

One skilled in the art will appreciate that purification methods suitable for native DAF may require modification to account for changes in the character of DAF or its variants upon expression in recombinant cell culture. For example, a DAF polypeptide produced in prokaryotic cell culture will not adsorb to Con-A Sepharose because it will be unglycosylated. In this case, other methods such as gel electrophoresis, ion exchange or immunoaffinity purification should be employed. Similarly, sDAF lipid-free C-terminal mDAF variants will not adsorb as readily to hydrophobic adsorbents as does mDAF. Appropriate purification methods will be apparent to the artisan, depending upon the characteristics of the particular recombinant DAF.

DAF is prepared as a nontoxic salt with such ions as sodium, potassium, phosphate, chloride and the like. Generally, DAF is stored in phosphate buffered saline or may be lyophilized in the present of an excipient including sugar alcohols, e.g. mannitol or sorbitol; monosaccharides, e.g., glucose, mannose, galactose or fructose; oligosaccharides such as maltose, lactose or sucrose; and proteins such as human serum albumin.

The foregoing excipients also may contribute to the stability of DAF to inactivation or precipitation upon aqueous storage, and may be used together with other stabilizers which are conventional per se. Such stabilizers include chelating agents, e.g. EDTA; antioxidants such as ascorbate or dithiothreitol; amino acids; and nonionic surfactants such as polyethylene glycol or block copolymers of polyethylene and polypropylene glycol.

DAF is administered to humans or animals in order to ameliorate various disorders stemming from immune dysfunction or misdirection, particularly defects in the humoral immune response. Examples include PNH, inflammatory conditions such as inflammatory bowel disease (colitis), rheumatoid arthritis, allograft rejection and the like. Treatment with DAF should be instituted early in the development of such disorders.

Therapeutic DAF compositions will contain a therapeutically effective dose of DAF in a pharmacologically acceptable carrier. The dose, carrier and route of administration selected will depend, among other factors, upon the disorder or condition to be treated, the condition of the patient, the desired route of administration, and the activity of the selected DAF variant. This is readily determined and monitored by the physician during the course of therapy.

The carrier for infusion or injection of DAF is a sterile isotonic aqueous solution, for example saline for injection or 5% dextrose. These preparations are injected or infused by intranasal, subcutaneous, intravenous, intraperitoneal or other conventional routes of adminstration. Preparations also are injected into the synonial fluid of arthritic joints.

DAF also is provided in a sustained release carrier. Suitable examples include semipermeable polymer matrices in the form of shaped articles, e.g. suppositories, or microcapsules. Implantable or microcapsules sustained release matrices include polylactides (U.S. Patent 3,773,919, EP 58,481) copolymers of L-glutamic acid and gamma ethyl-L-glutamate (U. Sidman et al., 1983, "Biopolymers" 22(1): 547-556), poly (2-hydroxyethyl-methacrylate) (R. Langer et al., 1981, "J. Biomed. Mater. Res." 15: 167-277 and R. Langer, 1982, "Chem. Tech." 12: 98-105), ethylene vinyl acetate (R. Langer et al., Id.), or poly-D-(-)-3-Hydroxybutyric acid (EP 133,988A). Sustained release DAF compositions also include liposomally entrapped DAF. Liposomes containing DAF are prepared by methods known per se: DE 3,218,121A; Epstein et al.. 1985, "Proc. Natl. Acad. Sci. USA" 82: 3688-3692; Hwang et al., 1980, "Proc. Natl. Acad. Sci. USA" 77: 4030-4034; EP 52322A; EP 36676A; EP 88046A; EP 143949A; EP 142641A; Japanese patent application 83-118008; U.S. patents 4,485,045 and 4,544,545; and EP 102,324A. Ordinarily the liposomes are of the small (about 200-800 Angstroms) unilamelar type in which the lipid content is greater than about 30 mol. % cholesterol, the selected proportion being adjusted for the optimal rate of DAF leakage.

Sustained release DAF preparations are implanted or injected into proximity to the site of inflammation or therapy, for example adjacent to arthritic joints or inflamed intestinal tissue.

Polyclonal rabbit or murine antisera raised against DAF are described by Medof et al. (1984, Id.). Antisera are employed for immunoaffinity purification or DAF and in an ELISA assay for DAF. Antibody specific for the unique C-terminus of sDAF is made by immunizing an animal against an immunogenic sDAF conjugate, e.g. an immunogenic fusion made in recombinant cell culture as described elsewhere herein, and thereafter screening for the presence of anti-sDAF titer by passing the antiserum through a column of immobilized mDAF in order to adsorb antibodies directed against mDAF epitopes, incubating the unadsorbed antiserum in the presence of ¹²⁵I-sDAF (prepared in substantially the same fashion as ¹²⁵I-mDAF, Medof et al., 1984, Id.) to permit the unique sDAF epitopes to bind to the anti-sDAF antibodies in the unadsorbed antiserum, and determining the amount of unbound ¹²⁵I-sDAF, e.g. by adsorption on protein-A Sepharose.

The sDAF-specific antibodies in such antisera are prepared by adsorption as immobilized mDAF, recovery of the unadsorbed fraction, adsorption on immobilized sDAF and elution with pH 4-6 buffer to recover the sDAF-specific antibodies substantially free of mDAF antibodies. Alternatively, spleen cells from immunized animals showing anti-sDAF neutralizing titer are recovered and fused to myeloma cells or are transformed with EB virus in known fashion in order to prepare monoclonal sDAF-specific antibodies.

Neutralizing antibodies against DAF are useful when conjugated to immunogenic polypeptides as immunogens for raising anti-idiotypic antibodies having DAF activity. Such anti-idiotypic antibodies are useful for the same diagnostic and therapeutic purposes as DAF.

In order to simplify the Examples certain frequently occurring methods will be referenced by shorthand phrases.

"Plasmids" are designated by a low case p preceded and/or followed by capital letters and/or numbers. The starting plasmids herein are commercially available, are publicly available on an unrestricted basis, or can be constructed from such available plasmids in accord with published procedures. In addition, other equivalent plasmids are known in the art and will be apparent to the ordinary artisan.

"Digestion" of DNA refers to catalytic cleavage of the DNA with an enzyme that acts only at certain locations in the DNA. Such enzymes are called restriction enzymes, and the sites for which each is specific is called a restriction site. The various restriction enzymes used herein are commercially available and their reaction conditons, cofactors and other requirements as established by the enzyme suppliers were used. Restriction enzymes commonly are designated by abbreviations composed of a capital letter followed by other letters representing the microorganism from which each restriction enzyme originally was obtained and then a number designating the particular enzyme. In general, about 1 µg of plasmid or DNA fragment is used with about 2 units of enzyme in about 20 µl of buffer solution. Appropriate buffers and substrate amounts for particular restriction enzymes are specified by the manufacturer. Incubation times of about 1 hour at 37°C are ordinarily used, but may vary in accordance with the supplier's instructions. After incubation, protein is removed by extraction with phenol and chloroform, and the digested nucleic acid is recovered from the aqeuous fraction by precipitation with ethanol. Digestion with a restriction enzyme infrequently is followed with bacterial alkaline phosphatase hydrolysis of the terminal 5' phosphates to prevent the two restriction cleaved ends of a DNA fragment from "circularizing" or forming a closed loop that would impede insertion of another DNA fragment at the restriction site. Unless otherwise stated, digestion of plasmids is not followed by 5' terminal dephosphorylation. Procedures and reagents for dephosphorylation are conventional (T. Maniatis et al., 1982, Molecular Cloning pp. 133-134).

"Filling" or "blunting" refers to the procedure by which the single stranded end in the cohesive terminus of a restriction enzyme-cleaved nucleic acid is converted to a double strand. This eliminates the cohesive terminus and forms a blunt end. This process is a versatile tool for converting a restriction cut end that may be cohesive with the ends created by only one or a few other restriction enzymes into a terminus compatible with any blunt-cutting restriction endonuclease or other filled cohesive terminus. Typically, blunting is accomplished by incubating 2-15µg of the target DNA in 10mM Mg Cl₂, 1mM dithiothreitol, 50mM NaCl, 10mM Tris (pH 7.5) buffer at about 37°C in the presence of 8 units of the Klenow fragment of DNA polymerase I and 250 µM of each of the four deoxynucleoside triphosphates. The incubation generally is terminated after 30 min. by phenol and chloroform extraction and ethanol precipitation.

"Recovery" or "isolation" of a given fragment of DNA from a restriction digest means separation of the digest on polyacrylamide or agarose gel by electrophoresis, identification of the fragment of interest by comparison of its mobility versus that of marker DNA fragments of known molecular weight, removal of the gel section containing the desired fragment, and separation of the gel from DNA. This procedure is known generally. For example, see R. Lawn et al., 1981, "Nucleic Acids Res." 9:6103-6114, and D. Goeddel et al., 1980, "Nucleic Acids Res.: 8:4057.

"Northern" blotting is a method by which the presence of a cellular mRNA is confirmed by hybridization to a known, labelled oligonucleotide or DNA fragment. For the purposes herein, unless otherwise provided, Northern analysis shall mean electrophoretic separation of the mRNA on 1 percent agarose in the presence of a denaturant (formaldehyde ^{~}7%), transfer to nitrocellulose hybridization to the labelled fragment as described by T. Maniatis et al., Id., p. 202.

"Transformation" means introducing DNA into an organism so that the DNA is replicable, either as an extrachromosomal element or chromosomal integrant. Unless otherwise provided, the method used herein for transformation of E. coli is the CaCl₂ method of Mandel et al., 1970, "J. Mol. Biol." 53: 154.

"Ligation" refers to the process of forming phosphodiester bonds between two double stranded nucleic acid fragments (T. Maniatis et al., Id., p. 146). Unless otherwise provided, ligation may be accomplished using known buffers and conditions with 10 units of T4 DNA ligase ("ligase") per 0.5 µg of approximately equimolar amounts of the DNA fragments to be ligated.

"Preparation" of DNA from transformants means isolating plasmid DNA from microbial culture. Unless otherwise provided, the alkaline/SDS method of Maniatis et al., Id., P. 90, may be used.

"Oligonucleotides" are short length single or double stranded polydeoxynucleotides which are chemically synthesized by known methods and then purified on polyacrylamide gels.

The following examples are intended to merely illustrate the best mode now known for practicing the invention, but the invention is not to be considered limited thereto.

All literature citations herein are expressly incorporated by reference.

### Example 1

### Identification of cDNA clones encoding DAF Cloning of human DAF

- Human DAF was purified to homogeneity and 23 amino acids of N-terminal sequence were determined. Five of these were ambiguous.

A 69mer oligonucleotide probe based on this amino acid sequence was synthesized in vitro: The 32p-labelled (Kinased) probe had the following nucleotide sequence:

GCTGAGCACCTGCCCCCTGATGTGCCCAATGCCCAGCCTGCCCTGGAGGGCAAGAAACCCTTCC-
CTG

A Hela cell λ cDNA library (approx. 1 x 10⁶ recombinants) was screened under low stringency conditions with this 69mer. Only one DAF clone (λ21) was identified, together with 6 false positives (by sequencing, these turned out to have limited nucleic acid homology with the probe, but a totally different amino and sequence). λ21 contained an insert encoding the sequence: Asp.Cys.Gly.Leu.Pro.Pro.Asp.Val.Pro.Asn.Ala.Gln.Pro.Ala.Leu.Glu. Gly Arg.Thr.Ser.Ple.Pro.Gly., wherein the underlined residues differed from those identified by amino terminal sequencing.

The initial DAF clone (clone λ21) was 1395 bp in length and contained a poly A tail but was missing the initiator methionine.

To determine the size of DAF MRNA a Northern bolt containing Hela cell Poly A+ RNA was screened 32p-labelled with DAF λ21. This probe hybridized to two messages of sizes approximately 1500bp and 2,000 bp. These were of roughly equal intensity.

To identify longer DAF clones with extensions at either of the 5' or 3' ends, we isolated 2 small restriction fragments from the 5' and 3' ends of λ21 as follows:

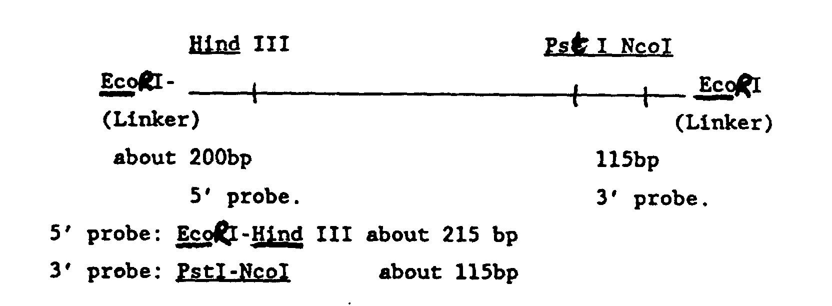
Hind III                                    Pst I NcoI
EcoRI-   EcoRI
(Linker)                                                    (Linker)
about 200bp                                    115bp
5' probe.                                      3' probe.
5' probe: EcoRI-Hind III about 215 bp
3' probe: PstI-NcoI      about 115bp

These probes were labelled with ³²p and used to rescreen the Hela CDNA library for additional DAF - encoding clones. 2 more clones were identified, DAF λ41 and DAF λ47. These hybridized to both probes and were longer than the DAF λ21 insert at approximately 2,000 bp and 2,200 bp respectively. Both of these clones contained about 780 bp of additional 3' untranslated sequence before the poly A tail. The 3'-untranslated sequence of the DAF gene contains a number of polyadenylation signals (AATAAA) and it appears that either an upstream of a downstream signal can be used to generate either the approx. 1,500 bp or the approx. 2,000 bp MRNAS.

At the 5' end, clone DAF λ41 was 55 bp longer than DAF λ21 and included an ATG for translation initiation. Clone DAF λ47 was 93 bp shorter than DAF λ21 at the 5' end.

Clone DAF 33 also was identified, but it only hybridized to the 5' probe. This clone was 71 bp longer than DAF λ21 at the 5' end, and therefore represented the longest extension in the 5' direction.

DAF λ21 and DAF λ41 were completely overlapping in the coding region of the protein and encoded a protein of 440 amino acids. DAF λ47 and DAF λ33 contained an apparent 'deletion' of 118 bp of coding region with respect to DAF λ21 and DAF λ41. On closer inspection it appeared that DAF λ21 and DAF λ41 contained an unspliced (unremoved) intron of 118 bp. Subsequently two more clones were identified, DAF λ35 and DAF λ37, one of which contains the same intron and one of which does not.

The frequency with which the unspliced form is present in the library (3 out of 6 clones) suggests that it is unlikely the unspliced clones represent improperly spliced message. Rather, there appear to be two forms of the DAF protein. These 2 forms are identical at amino acid positions 1-327, while having different C-terminal sequences. The unspliced form contains an additional 79 amino acids, the spliced form contains an additional 20 amino acids. Since the splice produces a change in reading frame there is no homology between the 2 proteins at the C-terminii.

From the hydropathy plots of the 2 DAF proteins, and from a comparison with the well-characterized Thy-1 membrane-bound glycoprotein, it is concluded that the spliced DAF cDNA directs synthesis of membrane-bound DAF, while the unspliced version encodes a soluble form.

### Example 2

### Expression of DAF In Recombinant Cell Culture

Clones DAF λ33, λ41 and λ47 from Example 1 were each subcloned into pUC19, a readily available cloning vector for E.coli, by digesting each of the λ clones with EcoRI, recovering the DAF inserts from each, digesting pUC19 with EcoRI, ligating the inserts into opened pUC19 and transforming E.coli 294 with the each ligation mixture. pUC1933, pUC1941 and pUC1947 were recovered from ampicillin resistant colonies.

pUC1933, pUC1941 and pUC1947 were each digested with EcoRI and HindIII and the fragments (I, II and III respectively) containing the 5' end of the DAF gene, and the 3' ends of the sDAF and mDAF genes, respectively, were recovered. pUC19 digested with EcoRI was ligated to Fragments I and II in a three way ligation and pUC19sDAF was recovered from an ampicillin resistant E.coli colony. This was the subclone of the complete sDAF gene shown in Figs 2a - 2c.

pUC19mDAF was constructed in the same way as pUC19sDAF except that Fragment III was used in place of Fragment II. This subclone contained the complete mDAF gene of Fig. 1a - 1c.

pE348HBVE400D22 (also pE342HBVE400D22, EP 117,058A) is digested with HindIII such that the DHFR - containing fragment is recovered. The HindIII cohesive terminii are filled, the fragment digested with Clal and the following fragment isolated

DHFR HBsAg Poly A pML SV40 ori
ClaI HindIII
(blunt)
(Fragment a, 4084 bp)

pE348 MBV E400D22 also is digested with ClaI and SocII such that the 990 bp fragment containing the SV40 ori and HBsAg poly A sequence is recovered (Fragment b).

pUCsDAF and pUCmDAF were digested with EcoRI and each DAF - encoding fragment isolated (Fragments CII and CIII, respectively).

Fragments CII, a and b are ligated in a three way ligation and transfected into E. coli 294. pE348sDAF is recovered from an ampicillin resistant colony. It contains the sDAF gene in proper orientation 3' to the SV40 sDAF early promoter. The sDAF gene is under the control of the SV40 early promoter in an expression vector suitable for transformation into and methotrexate selection and amplification in a mammalian host cell.

pE348mDAF is constructed in the same way except that Fragment CIII is used.

An alternative expression vector is constructed by digesting p342E (Crowley et al., 1983, "Mol. Cell. Biol." 3:44-55) with EcoRI and HpaI, and the vector fragment recovered. Either of pUC19mDAF or pUC19sDAF are digested with AccI (for mDAF) or blunt XhoII (for sDAF), filled, digested with EcoRI and the DAF-encoding fragments recovered. The DAF fragments are ligated into the vector fragment and expression vectors recovered. This vector does not contain the DHFR gene, although cotransformation with pFD11 (Simonsen et al., 1983, "P.N.A.S.-USA" 80:2495-99) will produce satisfactory results.

pE348mDAF or pE348sDAF are co-transfected into DHFR⁻ CHO cells using conventional methods, inoculated into HAT medium and transformants selected by culture in media containing serial increases in methotrexate concentration to amplify the DHFR and DAF genes. A transformant clone is recovered that stably expresses DAF and secretes it into the culture medium. The sDAF is recovered from the medium by adsorption onto an immunoaffinity column containing protein-A sepharose immobilized rabbit polyclonal antibody to sDAF and elution with pH5 glycine buffer.

pE348mDAF is transformed into an amplified in DHFR⁻CHO cells in the same way. mDAF is recovered by isolation from detergent lysates of host cell membranes in essentially the same fashion as mDAF has been recovered heretofore from red blood cell stroma.

## Claims

1. Nucleic acid which encodes mDAF as shown in Figure 1, sDAF as shown in Figure 2, or an amino acid sequence variant thereof wherein a predetermined amino acid residue or polypeptide is inserted into, deleted from or substituted for an amino acid residue or polypeptide of the native mature or preDAF amino acid sequence of mDAF shown in Figure 1 or sDAF shown in Figure 2, the variant being capable of exhibiting a biological activity in common with said mDAF or sDAF; which nucleic acid is (a) DNA free of an intron or (b) DNA free of flanking genomic DNA or (c) cell-free or (d) free of nucleic acid. encoding any other protein homologous to the source of the nucleic acid that encodes DAF.

2. Variant-encoding nucleic acid of claim 1 wherein the predetermined residue or polypeptide is inserted into the mature DAF amino acid sequence.

3. Variant-encoding nucleic acid of claim 2 wherein mature DAF is fused at its amino terminus to the carboxyl terminus of a secretory signal sequence heterologous to the DAF.

4. Variant-encoding nucleic acid of claim 3 wherein the signal sequence is the signal sequence of a viral polypeptide.

5. Variant-encoding nucleic acid of claim 4 wherein the viral polypeptide is the signal sequence for the herpes simplex gD protein.

6. Variant-encoding nucleic acid of claim 2 wherein the variant is met-mature DAF.

7. Variant-encoding nucleic acid of claim 2 wherein the mature DAF is fused at its amino or carboxyl terminus to an immunogenic polypeptide.

8. Variant-encoding nucleic acid of claim 7 wherein the immunogenic polypeptide is a microbial polypeptide.

9. Variant-encoding nucleic acid of claim 2 wherein the mature DAF is fused at its carboxyl terminus to a polypeptide capable of binding to a cell surface protein.

10. Variant-encoding nucleic acid of claim 9 wherein the polypeptide is an antibody or protein-binding fragment thereof.

11. Variant-encoding nucleic acid of claim 10 wherein the antibody is directed against an HLA antigen.

12. Variant-encoding nucleic acid of claim 1 wherein a predetermined amino acid residue or polypeptide is deleted from the mature DAF amino acid sequence.

13. Variant-encoding nucleic acid of claim 12 wherein the predetermined amino acid residue or polypeptide is located in the C-terminus of mDAF.

14. Variant-encoding nucleic acid of claim 1 wherein a lysinyl or arginyl residue is deleted or substituted.

15. Variant-encoding nucleic acid of claim 1 wherein a predetermined amino acid residue or polypeptide is substituted for an amino acid residue or polypeptide in the mature DAF amino acid sequence.

16. Variant-encoding nucleic acid of claim 15 wherein a single residue is substituted for an amino acid residue in the mature DAF amino acid sequence.

17. Variant-encoding nucleic acid of claim 16 wherein the residue substituted into DAF contains a hydrophobic side chain.

18. Variant-encoding nucleic acid of claim 17 wherein the residue is valyl, isoleucyl, leucyl, phenylalanyl or alanyl.

19. Variant-encoding nucleic acid of claim 16 wherein the residue substituted into DAF contains an electronegative side chain.

20. Variant-encoding nucleic acid of claim 19 wherein the residue is glutamyl or aspartyl.

21. Variant-encoding nucleic acid of claim 16 wherein the residue substituted into DAF contains an electropositive side chain.

22. Variant-encoding nucleic acid of claim 21 wherein the residue is lysyl, arginyl or histidyl.

23. Variant-encoding nucleic acid of claim 16 wherein the residue is tryptophanyl, prolyl, tyrosyl, methionyl, seryl or threonyl.

24. Variant-encoding nucleic acid of claim 16 wherein the residue is prolyl, cysteinyl or glycyl.

25. Variant-encoding nucleic acid of claim 1 wherein a lysyl or arginyl residue within a DAF dibasic dipeptide containing lysyl or arginyl residues is substituted by a glutamyl or histidyl residue.

26. Variant-encoding nucleic acid of claim 1 wherein the variant is a fusion of a DAF sequence with an immunogenic polypeptide, hormone or antibody.

27. Variant-encoding nucleic acid of claim 1 wherein the DAF is mDAF.

28. Variant-encoding nucleic acid of claim 1 wherein the DAF is sDAF.

29. sDAF, having the mature or pre-sequence of Figure 2, free of homologous cells and free of at least one homologous plasma component.

30. Nucleic acid, other than native mRNA or genomic DNA that encodes DAF, that is capable of hybridizing with nucleic acid encoding mDAF as shown in Figure 1, sDAF as shown in Figure 2 or a variant as defined in any one of claims 1 to 27.

31. Nucleic acid encoding mDAF as shown in Figure 1 from which a sequence comprising Cys₃₃₀ - Thr₃₄₇ is deleted.

32. A replicable vector comprising nucleic acid encoding mDAF as shown in Figure 1, sDAF as shown in Figure 2 or a variant as defined in any one of claims 1 to 27.

33. The vector of claim 32 comprising nucleic acid encoding sDAF.

34. A composition comprising a host cell transformed with the vector of claim 32 or 33.

35. The composition of claim 34 wherein the cell is a mammalian cell.

36. A method for making mDAF as shown in Figure 1, sDAF as shown in Figure 2 or a variant as defined in any one of claims 1 to 27, comprising culturing a host cell transformed with a vector containing nucleic acid encoding said mDAF, said sDAF, or a said variant operably linked to a promoter, under conditions for expressing mDAF, sDAF or a said variant.

37. The method of claim 36 wherein the host cell is a mammalian cell.

38. The method of claim 37 wherein the mDAF, sDAF or variant is recovered from the culture medium of the host cell.

39. The method of claim 38 wherein sDAF is recovered.

40. DAF, having the mature or pre-amino acid sequence of mDAF shown in Figure 1 or of sDAF as shown in Figure 2, unaccompanied by native glycosylation.

41. The DAF of claim 40 which is unglycosylated.

42. DAF unaccompanied by native glycosylation as produced by the process of claim 36.

43. A pharmaceutical composition for inhibiting antibody-mediated rejection of allografts or for ameliorating auto-immune responses against target tissues which comprises an immunomodulatory conjugate of DAF, having the mature or pre-sequence of mDAF shown in Figure 1 or of sDAF shown in Figure 2, linked to a substance capable of specifically binding the allograft or target tissue, with a physiologically innocuous excipient.

44. The composition of claim 43 wherein the DAF is mDAF.

45. The composition of claim 43 wherein the substance is an antibody or fragment thereof capable of binding an HLA antigen present in the allograft that is not expressed by the graft recipient.

46. The composition of claim 45 wherein the antibody or fragment thereof is linked by a covalent bond.

47. The composition of claim 45 wherein the antibody or fragment thereof is fused to the C-terminus of sDAF as obtained by expression in a recombinant host-vector system.

48. A composition comprising an antibody capable of binding the mature or pre-sDAF polypeptide of Figure 2 which composition is essentially free of antibodies capable of binding the mature or pre-mDAF polypeptide of Figure 1.

49. The composition of claim 48 wherein the anti-sDAF antibody is a monoclonal antibody.

50. A polypeptide comprising a phospholipid anchor domain of mDAF, including a C-terminal domain of mDAF of amino acid sequence present in Figure 1, fused to a polypeptide other than DAF.

51. A polypeptide which is a fusion of (a) the C-terminal domain of mDAF comprising Cys, and residues 331-347 as shown in Figure 1 and (b) a polypeptide other than DAF.

52. A polypeptide according to claim 50 or 51 wherein said polypeptide other than DAF is a preprotein.

53. A polypeptide according to claim 50 or 51 wherein said polypeptide other than DAF is a hormone.

54. A polypeptide according to any one of claims 50 to 53 wherein said fusion is at the C-terminus of said polypeptide other than DAF.

## Patentansprüche

1. Nucleinsäure, die für den in Fig. 1 dargestellten mDAF, den in Fig. 2 dargestellten sDAF oder eine Aminosäuresequenzvariante davon kodiert, worin ein vorbestimmter(s) Aminosäurerest oder Polypeptid in die native reife oder Prä-DAF-Aminosäuresequenz des in Fig. 1 dargestellten mDAF oder in Fig. 2 dargestellten sDAF eingeführt ist, daraus deletiert ist oder einen Aminosäurerest oder ein Polypeptid davon substituiert, wobei die Variante fähig ist, eine biologische Aktivität aufzuweisen, die sie mit dem mDAF oder sDAF gemeinsam hat; wobei die Nucleinsäure (a) DNA, die frei von Introns ist, oder (b) DNA, die frei von flankierender genomischer DNA ist, oder (c) zellfrei oder (d) frei von Nucleinsäure ist, die für ein beliebiges anderes Protein kodiert, das homolog zur Quelle der für den DAF kodierenden Nucleinsäure ist.

2. Für eine Variante kodierende Nucleinsäure nach Anspruch 1, worin der vorbestimmte Rest oder das vorbestimmte Polypeptid in die reife DAF-Aminosäuresequenz eingeführt ist.

3. Für eine Variante kodierende Nucleinsäure nach Anspruch 2, worin reifer DAF an seinem Aminoterminus mit dem Carboxyterminus einer zum DAF heterologen Sekretionssignalsequenz fusioniert ist.

4. Für eine Variante kodierende Nucleinsäure nach Anspruch 3, worin die Signalsequenz die Signalsequenz eines viralen Polypeptids ist.

5. Für eine Variante kodierende Nucleinsäure nach Anspruch 4, worin das virale Polypeptid die Signalsequenz für das Herpes-simplex-gD-Protein ist.

6. Für eine Variante kodierende Nucleinsäure nach Anspruch 2, worin die Variante met-reifer DAF ist.

7. Für eine Variante kodierende Nucleinsäure nach Anspruch 2, worin der reife DAF an seinem Amino- oder Carboxyterminus mit einem immunogenen Polypeptid fusioniert ist.

8. Für eine Variante kodierende Nucleinsäure nach Anspruch 7, worin das immunogene Polypeptid ein mikrobielles Polypeptid ist.

9. Für eine Variante kodierende Nucleinsäure nach Anspruch 2, worin der reife DAF an seinem Carboxyterminus mit einem Polypeptid fusioniert ist, das an ein Zelloberflächenprotein binden kann.

10. Für eine Variante kodierende Nucleinsäure nach Anspruch 9, worin das Polypeptid ein Antikörper oder proteinbindendes Fragment davon ist.

11. Für eine Variante kodierende Nucleinsäure nach Anspruch 10, worin der Antikörper gegen ein HLA-Antigen gerichtet ist.

12. Für eine Variante kodierende Nucleinsäure nach Anspruch 1, worin ein vorbestimmter(s) Aminosäurerest oder Polypeptid aus der reifen DAF-Aminosäuresequenz deletiert ist.

13. Für eine Variante kodierende Nucleinsäure nach Anspruch 12, worin sich der vorbestimmte Aminosäurerest oder das vorbestimmte Polypeptid am C-Terminus vom mDAF befindet.

14. Für eine Variante kodierende Nucleinsäure nach Anspruch 1, worin ein Lysinyloder Arginylrest deletiert oder substituiert ist.

15. Für eine Variante kodierende Nucleinsäure nach Anspruch 1, worin ein vorbestimmter(s) Aminosäurerest oder Polypeptid einen Aminosäurerest oder ein Polypeptid in der reifen DAF-Aminosäuresequenz substituiert.

16. Für eine Variante kodierende Nucleinsäure nach Anspruch 15, worin ein einziger Rest einen Aminosäurerest in der reifen DAF-Aminosäuresequenz substituiert.

17. Für eine Variante kodierende Nucleinsäure nach Anspruch 16, worin der in DAF substituierte Rest eine hydrophobe Seitenkette enthält.

18. Für eine Variante kodierende Nucleinsäure nach Anspruch 17, worin der Rest Valyl, Isoleucyl, Leucyl, Phenylalanyl oder Alanyl ist.

19. Für eine Variante kodierende Nucleinsäure nach Anspruch 16, worin der in DAF substituierte Rest eine elektronegative Seitenkette enthält.

20. Für eine Variante kodierende Nucleinsäure nach Anspruch 19, worin der Rest Glutamyl oder Aspartyl ist.

21. Für eine Variante kodierende Nucleinsäure nach Anspruch 16, worin der in DAF substituierte Rest eine elektropositive Seitenkette enthält.

22. Für eine Variante kodierende Nucleinsäure nach Anspruch 21, worin der Rest Lysyl, Arginyl oder Histidyl ist.

23. Für eine Variante kodierende Nucleinsäure nach Anspruch 16, worin der Rest Tryptophanyl, Prolyl, Tyrosyl, Methionyl, Seryl oder Threonyl ist.

24. Für eine Variante kodierende Nucleinsäure nach Anspruch 16, worin der Rest Prolyl, Cysteinyl oder Glycidyl ist.

25. Für eine Variante kodierende Nucleinsäure nach Anspruch 1, worin ein Lysyloder Arginylrest innerhalb eines Lysyl- oder Arginylreste enthaltenden zweibasigen DAF-Dipeptids durch einen Glutamyl- oder Histidylrest substituiert ist.

26. Für eine Variante kodierende Nucleinsäure nach Anspruch 1, worin die Variante eine Fusion einer DAF-Sequenz mit einem immunogenen Polypeptid, Hormon oder Antikörper ist.

27. Für eine Variante kodierende Nucleinsäure nach Anspruch 1, worin der DAF mDAF ist.

28. Für eine Variante kodierende Nucleinsäure nach Anspruch 1, worin der DAF sDAF ist.

29. sDAF mit der reifen oder Prä-Sequenz von Fig. 2, frei von homologen Zellen und frei von zumindest einer homologen Plasmakomponente.

30. Nucleinsäure, mit Ausnahme von nativer mRNA oder genomischer DNA, die für DAF kodiert, die fähig ist, mit Nucleinsäure zu hybridisieren, die für den in Fig. 1 dargestellten mDAF, den in Fig. 2 dargestellten sDAF oder eine Variante nach einem der Ansprüche 1 bis 27 kodiert.

31. Nucleinsäure, die für den in Fig. 1 dargestellten mDAF kodiert, aus dem eine Cys₃₃₀ - Thr₃₄₇ umfassende Sequenz deletiert ist.

32. Replizierbarer Vektor, der die Nucleinsäure umfasst, die für den in Fig. 1 dargestellten mDAF, den in Fig. 2 dargestellten sDAF oder eine Variante nach einem der Ansprüche 1 bis 27 kodiert.

33. Vektor nach Anspruch 32, umfassend die für sDAF kodierende Nucleinsäure.

34. Zusammensetzung, umfassend eine mit dem Vektor nach Anspruch 32 oder 33 transformierte Wirtszelle.

35. Zusammensetzung nach Anspruch 34, worin die Zelle eine Säugetierzelle ist.

36. Verfahren zur Herstellung des in Fig. 1 dargestellten mDAF, des in Fig. 2 dargestellten sDAF oder einer Variante nach einem der Ansprüche 1 bis 27, umfassend das Kultivieren einer Wirtszelle, die mit einem Vektor transformiert ist, der die Nucleinsäure enthält, die für den mDAF, den sDAF oder eine operabel an einen Promotor gebundene Variante kodiert, unter Bedingungen zum Exprimieren von mDAF, sDAF oder einer Variante.

37. Verfahren nach Anspruch 36, worin die Wirtszelle eine Säugetierzelle ist.

38. Verfahren nach Anspruch 37, worin der mDAF, sDAF oder die Variante aus dem Kulturmedium der Wirtszelle gewonnen wird.

39. Verfahren nach Anspruch 38, worin sDAF gewonnen wird.

40. DAF, der die reife oder Prä-Aminosäuresequenz von in Fig. 1 dargestelltem mDAF oder von in Fig. 2 dargestelltem sDAF aufweist und von keiner nativen Glykosylierung begleitet ist.

41. DAF nach Anspruch 40, der unglykosyliert ist.

42. Von keiner nativen Glykosylierung begleiteter DAF, hergestellt durch das Verfahren nach Anspruch 36.

43. Pharmazeutische Zusammensetzung zum Hemmen einer Antikörper-vermittelten Abstoßung von Allotransplantaten oder zum Verbessern von Autoimmunreaktionen gegen Zielgewebe, die ein immunomodulatorisches Konjugat von DAF umfasst, das die reife oder die Prä-Sequenz von in Fig. 1 dargestelltem mDAF oder von in Fig. 2 dargestelltem sDAF aufweist, gebunden an eine Substanz, die fähig ist, das Allotransplantat oder das Zielgewebe spezifisch zu binden, mit einem physiologisch harmlosen Exzipienten.

44. Zusammensetzung nach Anspruch 43, worin der DAF mDAF ist.

45. Zusammensetzung nach Anspruch 43, worin die Substanz ein Antikörper oder Fragment davon ist, der/das fähig ist, ein im Allotransplantat vorhandenes HLA-Antigen zu binden, das durch den Transplantatrezipienten nicht exprimiert wird.

46. Zusammensetzung nach Anspruch 45, worin der Antikörper oder das Fragment davon durch eine kovalente Bindung gebunden ist.

47. Zusammensetzung nach Anspruch 45, worin der Antikörper oder das Fragment davon mit dem C-Terminus von sDAF fusioniert ist, erhalten durch Expression in einem rekombinanten Wirtsvektorsystem.

48. Zusammensetzung, umfassend einen Antikörper, der fähig ist, das reife oder Prä-sDAF-Polypeptid von Fig. 2 zu binden, wobei die Zusammensetzung im Wesentlichen frei von Antikörpern ist, die fähig sind, das reife oder Prä-mDAF-Polypeptid von Fig. 1 zu binden.

49. Zusammensetzung nach Anspruch 48, worin der Anti-sDAF-Antikörper ein monoklonaler Antikörper ist.

50. Polypeptid, umfassend eine Phospholipid-Ankerdomäne von mDAF, einschließlich einer C-terminalen Domäne von mDAF der in Fig. 1 vorhandenen Aminosäuresequenz, die mit einem anderen Polypeptid als DAF fusioniert ist.

51. Polypeptid, das eine Fusion der (a) C-terminalen Domäne von in Fig. 1 dargestelltem mDAF, der Cys und die Reste 331 - 347 umfasst, und (b) eines anderen Polypeptids als DAF ist.

52. Polypeptid nach Anspruch 50 oder 51, worin das andere Polypeptid als DAF ein Präprotein ist.

53. Polypeptid nach Anspruch 50 oder 51, worin das andere Polypeptid als DAF ein Hormon ist.

54. Polypeptid nach einem der Ansprüche 50 bis 53, worin sich die Fusion am C-Terminus des anderen Polypeptids als DAF befindet.

## Revendications

1. Acide nucléique qui code pour mDAF de la manière représentée sur la figure 1, pour sDAF de la manière représentée sur la figure 2, ou un de ses variants de séquence d'aminoacides dans lequel un résidu d'aminoacide ou polypeptide prédéterminé est inséré dans un, éliminé par délétion d'un ou substitué en remplacement d'un, résidu d'aminoacide ou polypeptide de la séquence d'aminoacides mature ou de preDAF naturelle du mDAF représenté sur la figure 1 ou sDAF représenté sur la figure 2, le variant étant capable de présenter une activité biologique en commun avec ledit mDAF ou sDAF ; acide nucléique qui (a) est un ADN dépourvu d'intron ou (b) un ADN dépourvu d'ADN génomique adjacent ou (c) acellulaire ou (d) dépourvu d'acide nucléique codant pour n'importe quelle autre protéine homologue à la source de l'acide nucléique qui code pour DAF.

2. Acide nucléique codant pour un variant suivant la revendication 1, dans lequel le résidu ou polypeptide prédéterminé est inséré dans la séquence d'aminoacides du DAF mature.

3. Acide nucléique codant pour un variant suivant la revendication 2, dans lequel le DAF mature est fusionné à son extrémité amino à l'extrémité carboxyle d'une séquence signal sécrétoire hétérologue au DAF.

4. Acide nucléique codant pour un variant suivant la revendication 3, dans lequel la séquence signal est la séquence signal d'un polypeptide viral.

5. Acide nucléique codant pour un variant suivant la revendication 4, dans lequel le polypeptide viral est la séquence signal pour la protéine gD d'herpès simplex.

6. Acide nucléique codant pour un variant suivant la revendication 2, dans lequel le variant est le met-DAF mature.

7. Acide nucléique codant pour un variant suivant la revendication 2, dans lequel le DAF mature est fusionné à son extrémité amino ou carboxyle à un polypeptide immunogène.

8. Acide nucléique codant pour un variant suivant la revendication 7, dans lequel le polypeptide immunogène est un polypeptide microbien.

9. Acide nucléique codant pour un variant suivant la revendication 2, dans lequel le DAF mature est fusionné à son extrémité carboxyle à un polypeptide capable de se lier à une protéine de surface cellulaire.

10. Acide nucléique codant pour un variant suivant la revendication 9, dans lequel le polypeptide est un anticorps ou un de ses fragments de liaison de protéine.

11. Acide nucléique codant pour un variant suivant la revendication 10, dans lequel l'anticorps est dirigé contre un antigène HLA.

12. Acide nucléique codant pour un variant suivant la revendication 1, dans lequel un résidu d'aminoacide ou polypeptide prédéterminé est éliminé par délétion de la séquence d'aminoacides du DAF mature.

13. Acide nucléique codant pour un variant suivant la revendication 12, dans lequel le résidu d'aminoacide ou polypeptide prédéterminé est situé dans l'extrémité C-terminale du mDAF.

14. Acide nucléique codant pour un variant suivant la revendication 1, dans lequel un résidu lysinyle ou arginyle est éliminé par délétion ou substitué.

15. Acide nucléique codant pour un variant suivant la revendication 1, dans lequel un résidu d'aminoacide ou polypeptide prédéterminé est substitué en remplacement d'un résidu d'aminoacide ou polypeptide dans la séquence d'aminoacides du DAF mature.

16. Acide nucléique codant pour un variant suivant la revendication 15, dans lequel un résidu unique est substitué en remplacement d'un résidu d'aminoacide dans la séquence d'aminoacides du DAF mature.

17. Acide nucléique codant pour un variant suivant la revendication 16, dans lequel le résidu substitué dans le DAF contient une chaîne latérale hydrophobe.

18. Acide nucléique codant pour un variant suivant la revendication 17, dans lequel le résidu est un résidu valyle, isoleucyle, leucyle, phénylalanyle ou alanyle.

19. Acide nucléique codant pour un variant suivant la revendication 16, dans lequel le résidu substitué dans le DAF contient une chaîne latérale électronégative.

20. Acide nucléique codant pour un variant suivant la revendication 19, dans lequel le résidu est un résidu glutamyle ou aspartyle.

21. Acide nucléique codant pour un variant suivant la revendication 16, dans lequel le résidu substitué dans le DAF contient une chaîne latérale électropositive.

22. Acide nucléique codant pour un variant suivant la revendication 21, dans lequel le résidu est un résidu lysyle, arginyle ou histidyle.

23. Acide nucléique codant pour un variant suivant la revendication 16, dans lequel le résidu est un résidu tryptophanyle, prolyle, tyrosyle, méthionyle, séryle ou thréonyle.

24. Acide nucléique codant pour un variant suivant la revendication 16, dans lequel le résidu est un résidu prolyle, cystéinyle ou glycyle.

25. Acide nucléique codant pour un variant suivant la revendication 1, dans lequel un résidu lysyle ou arginyle à l'intérieur d'un dipeptide dibasique DAF contenant des résidus lysyle ou arginyle est substitué par un résidu glutamyle ou histidyle.

26. Acide nucléique codant pour un variant suivant la revendication 1, dans lequel le variant est une fusion d'une séquence de DAF avec un polypeptide immunogène, une hormone ou un anticorps.

27. Acide nucléique codant pour un variant suivant la revendication 1, dans lequel le DAF est le mDAF.

28. Acide nucléique codant pour un variant suivant la revendication 1, dans lequel le DAF est le sDAF.

29. sDAF ayant la séquence mature ou préséquence de la figure 2, dépourvu de cellules homologues et dépourvu d'au moins un constituant plasmatique homologue.

30. Acide nucléique, autre que l'ARNm naturel ou ADN génomique qui code pour le DAF, qui est capable de s'hybrider avec l'acide nucléique codant pour le mDAF de la manière représentée sur la figure 1, le sDAF de la manière représentée sur la figure 2 ou un variant tel que défini dans l'une quelconque des revendications 1 à 27.

31. Acide nucléique codant pour le mDAF de la manière représentée sur la figure 1, duquel une séquence comprenant Cys₃₃₀ - Thr₃₄₇ a été éliminée par délétion.

32. Vecteur réplicable comprenant l'acide nucléique codant pour le mDAF de la manière représentée sur la figure 1, sDAF de la manière représentée sur la figure 2 ou un variant tel que défini dans l'une quelconque des revendications 1 à 27.

33. Vecteur suivant la revendication 32 comprenant l'acide nucléique codant pour le sDAF.

34. Composition comprenant une cellule hôte transformée avec le vecteur de la revendication 32 ou 33.

35. Composition suivant la revendication 34, dans laquelle la cellule est une cellule de mammifère.

36. Procédé pour la préparation du mDAF de la manière représentée sur la figure 1, du sDAF de la manière représentée sur la figure 2 ou d'un variant tel que défini dans l'une quelconque des revendications 1 à 27, comprenant la culture d'une cellule hôte transformée avec un vecteur contenant l'acide nucléique codant pour ledit mDAF, ledit sDAF ou un tel variant lié de manière fonctionnelle à un promoteur, dans des conditions pour l'expression du mDAF, du sDAF ou d'un tel variant.

37. Procédé suivant la revendication 36, dans lequel la cellule hôte est une cellule de mammifère.

38. Procédé suivant la revendication 37, dans lequel le mDAF, le sDAF ou le variant est recueilli à partir du milieu de culture de la cellule hôte.

39. Procédé suivant la revendication 38, dans lequel le sDAF est recueilli.

40. DAF ayant la séquence mature ou préséquence d'aminoacides du mDAF représenté sur la figure 1 ou du sDAF représenté sur la figure 2, non accompagné par la glycosylation naturelle.

41. DAF suivant la revendication 40, qui est non glycosylé.

42. DAF non accompagné par la glycosylation naturelle, tel que produit par le procédé de la revendication 36.

43. Composition pharmaceutique pour inhiber le rejet à médiation par anticorps, d'allogreffes ou pour améliorer les réponses autoimmunes contre des tissus cibles, qui comprend un conjugué immunomodulateur de DAF, ayant la séquence mature ou préséquence du mDAF représenté sur la figure 1 ou du sDAF représenté sur la figure 2, lié à une substance capable de lier spécifiquement l'allogreffe ou le tissu cible, avec un excipient physiologiquement non nocif.

44. Composition suivant la revendication 43, dans laquelle le DAF est le mDAF.

45. Composition suivant la revendication 43, dans lequel la substance est un anticorps ou un de ses fragments capable de se lier à un antigène HLA présent dans l'allogreffe qui n'est pas exprimé par le receveur de la greffe.

46. Composition suivant la revendication 45, dans laquelle l'anticorps ou son fragment est lié par une liaison covalente.

47. Composition suivant la revendication 45, dans laquelle l'anticorps ou son fragment est fusionné à l'extrémité C-terminale du sDAF tel qu'obtenu par expression dans un système hôte-vecteur recombinant.

48. Composition comprenant un anticorps capable de se lier au polypeptide sDAF mature ou prépolypeptide sDAF de la figure 2, composition qui est pratiquement dépourvue d'anticorps capables de se lier au polypeptide sDAF mature ou prépolypeptide mDAF de la figure 1.

49. Composition suivant la revendication 48, dans laquelle l'anti-sDAF est un anticorps monoclonal.

50. Polypeptide comprenant un domaine d'ancrage aux phospholipides du mDAF, comprenant un domaine C-terminal du mDAF ayant la séquence d'aminoacides représentée sur la figure 1, fusionné à un polypeptide autre que le DAF.

51. Polypeptide qui est une fusion (a) du domaine C-terminal du mDAF comprenant Cys₃₃₀ et les résidus 331-347 de la manière représentée sur la figure 1 et (b) un polypeptide autre que DAF.

52. Polypeptide suivant la revendication 50 ou 51, dans lequel ledit polypeptide autre que le DAF est une préprotéine.

53. Polypeptide suivant la revendication 50 ou 51, dans lequel ledit polypeptide autre que le DAF est une hormone.

54. Polypeptide suivant l'une quelconque des revendications 50 à 53, dans lequel ladite fusion est à l'extrémité C-terminale dudit polypeptide autre que le DAF.
